# EUROPEAN PATENT APPLICATION

(11) **EP 4 309 663 A1**
(43) Date of publication of application: **24.01.2024**
(21) Application number: 22185858.2
(22) Date of filing: 19.07.2022
(51) Int. Cl.: A61K 35/74, A23L 33/135, A61P 1/00, A61P 29/00, C12N 1/20

(54) **MICROORGANISMS SYNTHESIZING ANTI-INFLAMMATORY MOLECULES**

(71) Applicant: Enbiosis Biotechnology Limited, Leicester LE1 1LB (GB)
(72) Inventor: Nalbantoglu, Özkan Ufuk, Izmir (TR); Özkan, Ömer, ISTANBUL (TR); Aycan, Gündogdu, Ankara (TR)
(74) Representative: Sach, Greg Robert

(57) **Abstract**

The present invention relates to the field of prevention and/or treatment of Inflammatory Bowel Diseases such as Crohn's Disease and Ulcerative Colitis.

## Description

### TECHNICAL FIELD

The present invention relates to the field of gastro-intestinal health and/or prevention and/or treatment of Inflammatory Bowel Diseases such as Crohn's Disease and Ulcerative Colitis.

### BACKGROUND ART

Inflammatory bowel disease includes disorders associated with chronic inflammation in the human intestine. These diseases often manifest as Crohn's Disease or Ulcerative Colitis. Crohn's disease causes pain and swelling in the digestive tract. It can affect anywhere from the mouth to the anus. It most commonly affects the small intestine and upper part of the large intestine. Ulcerative colitis causes swelling and sores (ulcers) in the large intestine (colon and rectum). Microscopic colitis causes intestinal inflammation that can only be detected with a microscope.

Drugs used to treat ulcerative colitis or Crohn's disease in the state of the art include:
- aminosalicylates or mesalazines, which can reduce inflammation in the intestine
- steroids or immunosuppressants, such as azathioprine, to reduce the activity of the immune system
- biologic drugs and treatments
- injectable antibody-based therapies that target a specific part of the immune system
- antibiotics

It is the generally accepted etiological view that chronic inflammation occurs as a result of an autoimmune phenomenon (Wen and Fiocchi, 2004). It is known that the gut microbiome changes in the case of disease and the disease is associated with the gut microbiome (Khan et al., 2019).

It has been suggested that a significant part of the immune system is located in the intestinal epithelium, is trained by commensal intestinal microorganisms throughout life, and that immunomodulations occur to a great extent in this way, but the disruption of this symbiotic balance may be one of the main causes of the disease. (Pratt et al., 2021).

At this point, two molecules originating from the gut microbiome associated with immunomodulation and related inflammation have come to the fore in recent years. It has been observed that the Microbial Anti-Inflammatory Molecule (MAM) protein originating from Faecalibacterium prausnitzii spp. bacteria has an anti-inflammatory effect and inhibits pathogenic signals by suppressing the Nuclear Factor kappa-B pathway. In fact, it has been observed that this protein provides protection against colitis by producing it in situ in animal experiments (Breyner et al., 2017). On the other hand, it is known that the molecular structure of polysaccharide A, which is avaliable as a surface display molecule in Bacteroides fragilis spp. bacteria, stimulates T cells in the direction of anti-inflammation and increases the secretion of Interlukin-10. (Ramakrishna et al. 2019). Trying to increase the level of IL-10 is seen as one of the main targets in the treatment of inflammatory bowel disease (Li and He, 2004, Herfarth and Scholmerich, 2002). On the other hand, since the antigenic structures of polysaccharides can play an inflammation-stimulating role, induction of polysaccharide A may need to be supported through different balancing pathways.

Thus, there is a need a new treatment of Inflammatory Bowel Disease.

Although it has been reported in the literature that the Microbial Anti-Inflammatory Molecule (MAM) protein of Faecalibacterium prausnitzii bacteria origin and the polysaccharide A molecule present as surface display molecule in Bacteroides fragilis bacteria have anti-inflammatory activity, there has not been a method that will produce both products and have its synergetic effect in a single engineered microorganism. In the present invention, it has been found that the anti-inflammatory activity is higher in humans with microorganisms that can produce both molecules, and thus, a synthetic microorganism that can produce both molecules has been proposed.

There are studies in the literature on the effectiveness of oral administration of Polysaccharide A molecule, however, an approach for its application in situ by genetically modified organisms has not been identified.

### BRIEF DESCRIPTION OF THE INVENTION

The solution to the above need is described in the claims.

The present disclosure relates to microorganisms synthesizing the anti-inflammatory molecules developed for eliminating the aforementioned disadvantages and providing new advantages to the respective technical field.

The aim of the invention includes a gene encoding a protein with anti-inflammatory effect and microorganisms belonging to gene cluster synthesizing a polysaccharide molecule known to have anti-inflammatory effect, and their use for the prevention or treatment of Inflammatory Bowel Diseases such as Crohn's Disease and Ulcerative Colitis.

Specifically, the microorganism simultaneously possesses a gene encoding a specific protein at 15kDA weight known as the Microbial Anti-Inflammatory Molecule (MAM) and the gene pathway responsible for the biosynthesis of the polysaccharide molecule known as polysaccharide A.

In particular, the invention relates to a microorganism comprising a microbial anti-inflammatory molecule (MAM) gene and one or more gene(s) encoding polysaccharide A biosynthesis pathway for use in the prevention and/or treatment of Inflammatory Bowel Disease, characterized in that the one or more gene(s) encoding said metabolic pathway is selected from the group of genes encoding UpaY, UpaZ, flippase, galactopyranose mutase, glycosyltransferase, amino sugar synthetase, undecaprenyl-phosphate galactose phosphotransferase, protein disulfide isomerase and methyltransferase protein.

Preferably, the microorganism is bacteria, yeast or fungi. In one further preferred embodiment the bacteria, yeast or fungi is produced synthetically by genetic engineering and/or MAM gene and/or the gene(s) encoding polysaccharide A biosynthesis pathway is transferred in vitro into the bacteria, yeast or fungi.

Preferably, the Inflammatory Bowel Disease is Crohn's Disease or Ulcerative Colitis.

In one further embodiment, the gene encoding MAM gene has at least 70% sequence identity with the Seg ID. No. 1 and/ or one or more of the genes encoding the polysaccharide A biosynthesis pathway has at least 70% sequence identity with one or more sequence(s) selected from the group consisting of Seg ID. No. 3, 5, 7, 9, 11, 13, 15, 17, 19, 21, 23 and 25.

The microorganism may preferably be in a physiologically acceptable carrier composition. This composition may be a food composition, preferably a dairy product or a fermented dairy product, preferably a yogurt or a yogurt drink.

In another preferred embodiment, the composition is a pharmaceutical composition, preferably in solid dosage form, preferably in the form of a capsule, a tablet or a powder.

The composition may be present in lyophilized or microencapsulated form and/or be as a probiotic to be administered.

In another aspect of the invention, the invention relates to the use of the above-mentioned microorganism and the preferred embodiments to protection, restoration and/or improvement of gastrointestinal health. In particular, the present invention may be used to maintaining a healthy gut, having a beneficial physiological effect to maintaining a normal digestion, improving gut regularity, supporting healthy gut mobility, bowel movement and/or healthy stool frequency, stool consistency and/or form and/or reducing bloating.

In a further aspect, the invention relates to a method of treatment of Inflammatory Bowel Disease or to a method of protection, restoration and/or improvement of gastrointestinal health for a subject in need with the microorganism or composition according to the present invention. The mircroorganism or composition is administered to the subject or patient in need of.

If the aforementioned microorganism strains are provided to a human in sufficient amounts by topical or oral route, it is expected to survive and colonize, at least temporarily, in the gastrointestinal tract of said human. In this colonization situation, it is expected that the increased in situ production of MAM and polysaccharide A in the human body will have a positive effect on human health and have an effect on preventing or treating Inflammatory Bowel Diseases such as Crohn's Disease and Ulcerative Colitis.

In the present invention, it is shown that the synergistic combined use of these two immunomodulatory agents in the conducted in vitro experiments significantly increased the anti-inflammatory effect through different pathways, and that this could be an important strategy in the immunomodulation treatment for inflammatory bowel disease. The invention relates to genetically modified probiotic/biotherapeutic microorganisms capable of producing both factors in order to apply said combined therapy in situ.

### BRIEF DESCRIPTION OF THE FIGURE

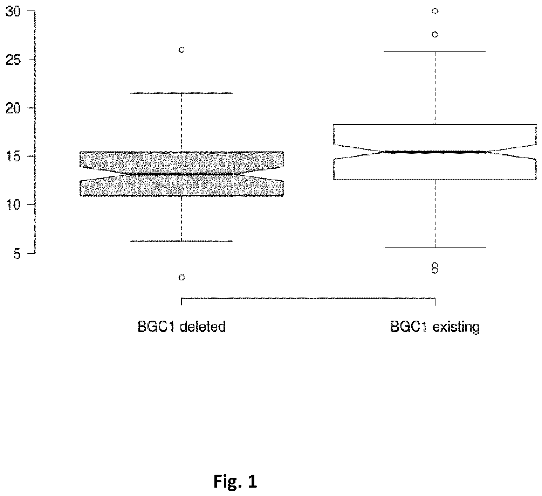

Figure 1: Plasma IL-10 levels (pg/ml) of the observed subgroups. People having the Microbial Anti-Inflammatory Molecule (MAM) gene and polysaccharide A biosynthetic gene cluster in their gut microbiome experience statistically significantly higher IL-10 secretion compared to the people lacking said pathway.

### DETAILED DESCRIPTION OF THE INVENTION

The preferred alternatives of the embodiments of invention, which are mentioned in this detailed description, are only intended for providing a better understanding of the subject-matter, and should not be construed in any restrictive sense.

### Microorganism

Primarily, the invention is directed to a microorganism comprising a gene encoding MAM and a gene cluster encoding the polysaccharide A biosynthesis pathway, which enables the microorganism to produce MAM and polysaccharide A molecules, preferablyunder anaerobic conditions. The microorganism may be an isolate belonging to the bacterial, archaeal, or fungal kingdoms.

The gene encoding the microbial anti-inflammatory molecule protein is the mam gene disclosed herein. The gene cluster belonging to the pathway synthesizing the polysaccharide A molecule must contain at least one of the genes encoding the following proteins: UpaY, UpaZ, flippase, galactopyranose mutase, glycosyltransferase, amino sugar synthetase, undecaprenyl-phosphate galactose phosphotransferase, protein disulfide isomerase, methyltransferase.

Said microorganism may also be a genetically modified bacterium, archaea or fungus, to which the above-mentioned genes have been transferred in vitro.

### Genes:

The gene encoding the microbial anti-inflammatory molecule has at least 70, 80, 90, 95, 99, 100% identity with Sequence ID No. 1. The respective amino acid sequence is shown in Sequence ID. No. 2.

The gene encoding the UpaY has at least 70, 80, 90, 95, 99, 100% identity with Sequence ID No. 3. The respective amino acid sequence is shown in Sequence ID. No. 4.

The gene encoding the UpaZ have at least 70, 80, 90, 95, 99, 100% identity with Sequence ID No. 5. The respective amino acid sequence is shown in Sequence ID. No. 6.

The gene encoding the flippase has at least 70, 80, 90, 95, 99, 100% identity with Sequence ID No. 7. The respective amino acid sequence is shown in Sequence ID. No. 8.

The gene encoding the galactopyranose mutase has at least 70, 80, 90, 95, 99, 100% identity with Sequence ID No. 9. The respective amino acid sequence is shown in Sequence ID. No. 10.

The gene encoding the glycosyltransferase must be a gene with at least 70, 80, 90, 95, 99, 100% identity with Sequence ID No. 11 or Sequence ID No. 13 or Sequence ID No. 15 or Sequence ID No. 17. The respective amino acid sequence are shown in Sequence ID. No. 12, 14, 16 and 18, respectively.

The gene encoding the amino sugar synthetase has at least 70, 80, 90, 95, 99, 100% identity with Sequence ID No. 19. The respective amino acid sequence is shown in Sequence ID. No. 20.

The gene encoding the undecaprenyl-phosphate galactose phosphotransferase has at least 70, 80, 90, 95, 99, 100% identity with Sequence ID No. 21. The respective amino acid sequence is shown in Sequence ID. No. 22.

The gene encoding the protein disulfide isomerase has at least 70, 80, 90, 95, 99, 100% identity with Sequence ID No. 23. The respective amino acid sequence is shown in Sequence ID. No. 24.

The gene encoding the methyltransferase has at least 70, 80, 90, 95, 99, 100% identity with Sequence ID No. 25. The respective amino acid sequence is shown in Sequence ID. No. 26.

### Composition:

The present invention also includes a composition comprising a bacterium according to the present disclosure and optionally a physiologically acceptable carrier. The physiologically acceptable carrier may be any suitable carrier for keeping the microorganism with said properties alive until it is consumed by a target organism (e.g., human or animal). For example, examples of acceptable carriers suitable for this purpose include any of the well known physiological or pharmaceutical carriers, buffers, and intermediates. The selection of an appropriate physiological or pharmaceutical carrier may vary depending on the intended mode of administration of the composition (e.g., oral) and the intended form of the composition (e.g., beverage, yogurt, powder, capsules, etc.) as discussed herein.

The composition containing said microorganisms may be in the form of food (food), feed, food formulation, food supplement composition or pharmaceutical composition.

Preferably said composition is a food or food supplement composition. The food or food supplement composition may be selected from the group consisting of a liquid, liquid beverage (including milk beverage and fermented beverage), yogurt, cheese, gel, gelatin, gelatin capsule, powder, paste, pressed tablet, and gel cap. The food or food supplement composition may be a dairy product, preferably a fermented dairy product, preferably a yogurt or a yogurt drink.

Preferably, said composition may be a probiotic composition. Such probiotic composition may include (isolated) bacteria, fungi, or a strain synthetically derived therefrom, as discussed herein. Preferably, the composition may further comprise one or more additional beneficial isolated strains of intestinal microorganism.

Preferably said composition may contain one or more prebiotic ingredients, any prebiotic ingredient suitable for increasing the activity of the microorganism and/or stimulating its growth. Examples of suitable prebiotic ingredients include fibers such as inulin, pectin, and resistant starch, as well as inositol, cellobiose, maltose, mannose, salicin, trehalose, amygdalin, arabinose, melibiose, sorbitol, rhamnose and/or xylose.

Said microorganism may be contained in lyophilized form, microencapsulated form (see for exampleSolanki et al., BioMed Res. Int. 2013, Article ID 620719), or any other form that protects the viability and activity of the microorganism species.

Preferably, said composition may be a pharmaceutical composition. The pharmaceutical composition may be produced for use as a supplement. A pharmaceutical composition will generally contain a pharmaceutical carrier in addition to the microorganism as mentioned herein. The carrier is preferably an inactive carrier. The preferred form depends on the intended mode of administration and (therapeutic) application. A pharmaceutical carrier may be any compatible, nontoxic substance suitable for delivering the organism mentioned herein into the body of a subject. For example, sterile water or inactive solids can be used as a carrier, often complemented by a pharmaceutically acceptable adjuvant, buffering agent, dispersing agent, and the like. The pharmaceutical composition may be in liquid form (e.g., a stabilized bacterial suspension of said microorganism) or in solid form (e.g., powder of said lyophilized microorganism). When said microorganism is lyophilized, a cryoprotectant such as lactose, trehalose or glycogen can be used. For example, for oral administration, said microorganism may be administered in solid dosage forms such as capsules, tablets and powders containing lyophilized microorganism, or in liquid dosage forms such as syrups and suspensions. Said microorganism may be carried, for example, in lyophilized form, in capsules such as gelatin capsules, in the form of inactive ingredients and, for example, glucose, lactose, sucrose, mannitol, starch, cellulose or cellulose derivatives, magnesium stearate, stearic acid, sodium saccharin, talc, magnesium carbonate and the like.

With the invention, it is intended to be used for the protection, restoration and/or improvement of gastrointestinal system health in general and/or for the prevention and/or treatment of Inflammatory Bowel Diseases such as Crohn's Disease and Ulcerative Colitis. It is intended herein to observe the expected efficacy by administering an effective amount of said microorganism to said subject by said means.

The following example that follows further illustrates the invention but should not be construed to limit the scope of the invention in any way.

### EXAMPLE

The discovery of phenotypic effects of the putative gene cluster was conducted via an observational study. The observation cohort consisted of 314 individuals with inflammatory bowel disease (171 ulcerative colitis and 143 Crohn's Disease patients). Stool samples of each participant were collected using appropriate fecal sampling techniques at the baseline. At the same time window, biochemical and immunological monitoring of each individual from peripheral blood was conducted. The fecal samples were subject to total DNA isolation. In order to evaluate the gut microbiome, shotgun metagenome sequencing was performed. Raw DNA sequencing data were analyzed using bioinformatic techniques. Each metagenome sample were mapped to reference genomes of known gut microbes and the relative coverage of each genomic regions were estimated. Characterization of the metabolic pathways were achieved by using KEGG patways database. It was observed that a individuals harboring both Microbial Anti-Inflammatory Molecule (MAM) gene and polysaccharide A biosynthetic gene cluster have significantly more pronounced anti-inflammatory activity monitored based on plasma Inteleukin-10 levels (Figure 1). The plasma IL-10 level of each individual was compared based on harboring the defined gene cluster. The observation cohort was divided into two subgroups which are the gene cluster deleted group (the genomic region containing the gene/pathway has less than 5% coverage of the rest of the genome) and the gene cluster harboring group. It was observed that the gene cluster deletion group experiences Ilower level of IL-10 compared to the people having a gut microbiome that contains the genes (p < 10⁻⁵, Mann-Whitney U-test).

Based on the experiments conducted in this cohort, it is hypothesized that people harboring microorganisms that are able to the corresponding inti-inflammatory molecules in their gut microbiome are in a trend of having more pronounced anti-inflammatory indicators. In case that organisms having that function are administired in a appropriate way, they can provide health benefits to the recepient experiencing pro-inflammation in inflammatory bowel disease. Furthermore, organisms with that property can be positioned as probiotics/biotherapeutics for prevention and treatment of inflammatory bowel diseases.

### References:

Wen Z, Fiocchi C. Inflammatory bowel disease: autoimmune or immune-mediated pathogenesis?. Clinical and Developmental Immunology. 2004 Sep 1;11(3-4): 195-204.
Khan I, Ullah N, Zha L, Bai Y, Khan A, Zhao T, Che T, Zhang C. Alteration of gut microbiota in inflammatory bowel disease (IBD): cause or consequence? IBD treatment targeting the gut microbiome. Pathogens. 2019 Sep; 8(3):126.
Pratt M, Forbes JD, Knox NC, Bernstein CN, Van Domselaar G. Microbiome-Mediated Immune Signaling in Inflammatory Bowel Disease and Colorectal Cancer: Support From Meta-omics Data. Frontiers in Cell and Developmental Biology. 2021; 9.
Breyner NM, Michon C, de Sousa CS, Vilas Boas PB, Chain F, Azevedo VA, Langella P, Chatel JM. Microbial anti-inflammatory molecule (MAM) from Faecalibacterium prausnitzii shows a protective effect on DNBS and DSS-induced colitis model in mice through inhibition of NF-κB pathway. Frontiers in microbiology. 2017 Feb 1; 8:114.
Ramakrishna C, Kujawski M, Chu H, Li L, Mazmanian SK, Cantin EM. Bacteroides fragilis polysaccharide A induces IL-10 secreting B and T cells that prevent viral encephalitis. Nature communications. 2019 May 14; 10(1):1-3.
Li MC, He SH. IL-10 and its related cytokines for treatment of inflammatory bowel disease. World Journal of Gastroenterology. 2004 Mar 1; 10(5):620.
Herfarth H, Scholmerich J. IL-10 therapy in Crohn's disease: at the crossroads. Gut. 2002 Feb 1; 50(2):146-7.
Solanki HK, Pawar DD, Shah DA, Prajapati VD, Jani GK, Mulla AM, Thakar PM. Development of microencapsulation delivery system for long-term preservation of probiotics as biotherapeutics agent. BioMed research international. Jan 2013 1; 2013.

## Claims

1. Microorganism comprising a microbial anti-inflammatory molecule (MAM) gene and one or more gene(s) encoding polysaccharide A biosynthesis pathway for use in the prevention and/or treatment of Inflammatory Bowel Diseases, **characterized in that**, one or more gene(s) encoding said metabolic pathway is selected from the group consisting of one or more of the genes encoding UpaY, UpaZ, flippase, galactopyranose mutase, glycosyltransferase, amino sugar synthetase, undecaprenyl-phosphate galactose phosphotransferase, protein disulfide isomerase and methyltransferase protein.

2. The microorganism according to claim 1, wherein the microorganism is bacteria, yeast or fungi.

3. The microorganism of claim 2, wherein the bacteria, yeast or fungi is produced synthetically by genetic engineering.

4. The microorganism of claim 2 or 3, wherein the MAM gene or the one or more gene(s) encoding polysaccharide A biosynthesis pathway is transferred in vitro into the bacteria, yeast or fungi.

5. The microorganism of any one of the preceding claims, wherein the Inflammatory Bowel Disease is Crohn's Disease or Ulcerative Colitis.

6. The microorganism of any one of the preceding claims, wherein the gene encoding MAM gene has at least 70% sequence identity with the Seg ID. No. 1.

7. The microorganism of any one of the preceding claims, wherein one or more of the genes encoding the polysaccharide A biosynthesis pathway has at least 70% sequence identity with one or more sequence(s) selected from the group consisting of Seg ID. No. 3, 5, 7, 9, 11, 13, 15, 17, 19, 21, 23 and 25.

8. The microorganism according to any one of the preceding claims, wherein it is in a physiologically acceptable carrier composition.

9. The composition according to claim 8, wherein it is a food composition, preferably a dairy product or a fermented dairy product, preferably a yogurt or a yogurt drink.

10. The composition according to claim 8, wherein it is a pharmaceutical composition, preferably in solid dosage form, preferably in the form of a capsule, a tablet or a powder.

11. The composition according to any one of claims 8-10, wherein the microorganism is present in the composition in lyophilized or microencapsulated form.

12. The composition according to any of the preceding claims as a probiotic to be administered.
